# EUROPÄISCHE PATENTSCHRIFT

(11) **EP 1 634 543 B1**
(45) Veröffentlichungstag und Bekanntmachung des Hinweises auf die Patenterteilung: **19.03.2008**
(21) Anmeldenummer: 04077524.9
(22) Anmeldetag: 09.09.2004
(51) Int. Cl.: A61B 19/00, A61B 17/34

(54) **Chirurgisches Instrument**
Surgical Instrument
Instrument de chirurgie

(43) Veröffentlichungstag der Anmeldung: 15.03.2006
(73) Patentinhaber: SOMATEX Medical Technologies GmbH, 14513 Teltow (DE)
(72) Erfinder: Kniep, Frank, 14979 Grossbeeren (DE); Hornscheidt, Dirk, 10245 Berlin (DE)
(74) Vertreter: Eisenführ, Speiser & Partner

(56) Entgegenhaltungen:
- EP-A1- 0 481 685
- EP-A1- 0 491 547
- DE-A1- 4 424 394
- US-A- 6 053 925
- US-A1- 2001 031 950

## Beschreibung

Die Erfindung betrifft ein chirurgisches Instrument, insbesondere ein Markierungsinstrument zum Markieren von Körpergewebeabschnitten, insbesondere von Tumorgewebe.

Derartige Markierungsinstrumente sind grundsätzlich bekannt, beispielsweise aus der DE 44 24 394 A1. In dieser Veröffentlichung ist auch die Verwendung derartiger Markierungsinstrumente beschrieben.

Das hier betroffene chirurgische Instrument besitzt eine Hohlnadel zum Einstechen in Körpergewebe. Die Hohlnadel schließt ein am distalen Ende der Hohlnadel und in der Regel auch am proximalen Ende der Hohlnadel offenes Lumen ein. Das Lumen ist in radialer Richtung durch eine Innenwand begrenzt.

Außerdem besitzt das hier betroffene chirurgische Instrument ein bezüglich der Hohlnadel längsverschiebliches Element.

Dieses Element kann beispielsweise ein radioaktiver Körper sein, der beispielsweise zur lokalen Tumorbehandlung implantiert wird und auch als "Seed" bekannt ist.

Das längsverschiebliche Element kann aber auch ein längsverschieblicher Draht sein, der wenigstens einen biegeelastischen distalen Endabschnitt aufweist, der in einem gestreckten Zustand derart elastisch vorgespannt ist, dass er in einem wenigstens annähernd entspannten Zustand eine Krümmung annimmt.

Der längsverschiebliche Draht ist derart in dem Lumen der Hohlnadel geführt, dass der vorgekrümmte Endabschnitt des Drahtes wahlweise aus dem offenen distalen Ende des Lumens herauszuschieben ist und dabei seine aufgeprägte Krümmung annimmt oder in das Lumen zurückzuziehen ist und dabei wenigstens annähernd gestreckt wird.

Zum Markieren eines Gewebeabschnittes wird zunächst die Hohlnadel mit in die Hohlnadel zurückgezogenem Draht in das Gewebe eingestochen und anschließend der Draht in der Hohlnadel vorgeschoben, so dass sein im gestreckten Zustand vorgespanntes distales Ende distal aus der Hohlnadel heraustritt. Aufgrund der Vorspannung des Drahtes biegt sich sein jeweiliger distaler Endabschnitt, wenn er aus dem distalen Ende der Hohlnadel heraustritt und nimmt die durch die Vorspannung aufgeprägte Vorkrümmung an. Soll das chirurgische Instrument repositioniert oder entfernt werden, kann der Draht in die Hohlnadel zurückgezogen werden. Dabei werden die vorgekrümmten Endabschnitte innerhalb der Hohlnadel wieder gestreckt. Der Innendurchmesser des Lumens der Hohlnadel ist nicht wesentlich größer als der Außendurchmesser des Drahtes im gestreckten Zustand.

An ein Markierungsinstrument der beschriebenen Art werden eine Reihe von Anforderungen gestellt, die teilweise im Widerspruch zueinander stehen und zu denen eine einfache und zuverlässige Handhabung ebenso zählt wie der Wunsch, ein möglichst großes Gewebevolumen definiert markieren zu können. Zu den gewünschten Eigenschaften zählt, dass mit dem Markierungsinstrument eine exakte Positionierung möglich sein soll. Das Markierungsinstrument sowie der längsverschiebliche Draht sollen möglichst repositionierbar sein. Der Draht soll eine möglichst große Rückhaltekraft im Gewebe bewirken. Außerdem soll sich der Draht in der Hohlnadel möglichst leicht vorschieben lassen. Und der Außendurchmesser der Nadel soll möglichst ≤1 mm sein.

Aus dem Stand der Technik bekannte Markierungsinstrumente, wie dasjenige aus der DE 44 24 394, sind im Bezug auf diese Anforderungen, insbesondere die Geometrie des Markerdrahtes und eine zuverlässige Handhabung, verbesserungsbedürftig.

Der Erfindung liegt daher die Aufgabe zugrunde, ein verbessertes chirurgisches Instrument, insbesondere ein verbessertes Markierungsinstrument anzubieten.

Erfindungsgemäß wird diese Aufgabe durch ein chirurgisches Instrument der zuvor beschriebenen Art erfüllt, bei der die Innenwand des Lumens mindestens abschnittsweise und vorzugsweise wenigstens im Bereich des distalen Endes des Lumens eine Gleitbeschichtung aufweist, die ein biokompatibles, organisches Gleitmittel umfasst. Auf diese Weise kann die Geometrie des längsverschieblichen Drahtes beispielsweise hinsichtlich Drahtquerschnitt und Vorkrümmung im entspannten Zustand samt der daraus im gestreckten Zustand resultierenden Vorspannung in weiten Grenzen gewählt werden, ohne dass dem durch Vorgaben hinsichtlich der Handhabung, z.B. ein leichtes Vorschieben des Drahtes und insbesondere ein Vermeiden des Ausknickens eines proximal aus dem Lumen herausragenden Drahtendes beim Vorschieben, allzu enge Grenzen gesetzt sind.

Es hat sich nämlich herausgestellt, dass durch eine derartige, spezielle Gleitbeschichtung auch bei stark einem vorgekrümmten distalen Endabschnitt des Drahtes in Kombination mit einer jeweils geeigneter Steifigkeit und Elastizität des Drahtes, hohe Handhabungskräfte, die z.B. auch zum Ausknicken des Drahtes führen können, vermieden werden können.

Alternativ denkbare Varianten, wie beispielsweise eine Teflonbeschichtung des Drahtes haben sich als vergleichsweise weniger wirksam erwiesen.

Ein besonders geeignetes Gleitmittel ist ein langkettiges Wachs, das vorzugsweise einen Schmelzpunkt von über 50°C oder besser noch über 60°C besitzt. Ein solcher Schmelzpunkt des Gleitmittels erlaubt eine wünschenswerte Sterilisation des chirurgischen Instrumentes, die beispielsweise Temperaturen von 40°C bis 50°C mit sich bringt.

Der Draht besitzt vorzugsweise wenigstens zwei in unterschiedliche Richtungen vorgekrümmte distale Endabschnitte, die an ihrem jeweiligen distalen Ende vorzugsweise angespitzt sind, um nach dem Austreten aus der Hohlnadel leichter in das umgebende Gewebe eindringen zu können. Das jeweilige, distale Ende eines distalen Endabschnittes kann durch Anschleifen oder Anschneiden angespitzt sein und besitzt aufgrund des Anschleifens oder Anschneidens eine schräge Anschliff- bzw. Anschnittfläche, die im spitzen Winkel zur Längsachse des jeweiligen Endabschnittes verläuft. Ein geeigneter Winkel zwischen Anschliff- oder Anschnittfläche und der Längsachse des jeweiligen Endabschnittes ist beispielsweise 30°. Die Anschliff- oder Anschnittflächen sind dabei vorzugsweise so angeordnet, dass sie bei in das Lumen zurückgezogenem Draht nach innen weisen, also von der Innenwand des Lumens abgewandt sind.

Ein besonders geeignetes Material für den längsverschieblichen Draht ist ein superelastisches Material, insbesondere eine superelastische Metalllegierung wie Nitinol. Nitinol ist eine dem Fachmann als superelastisch bekannte Nickel-Titan-Legierung.

Im Bereich der vorgekrümmten Endabschnitte ist der längsverschiebliche Draht vorzugsweise in einem Bogen um wenigstens 180° gekrümmt, um auf diese Weise eine sichere Verankerung in dem zu markierenden Gewebe zu gewährleisten. Eine derartig weitgehende Vorkrümmung, die in das Lumen zurückgezogenen distalen Endabschnitten zu hohen Normalkräften zwischen Draht und Innenwand des Lumens führt, lässt sich erst in Verbindung mit der erfindungsgemäßen Gleitbeschichtung verwirklichen, ohne dass die Kräfte zum Vorschieben des Drahtes zu groß werden und im Extremfall gar die Gefahr des Ausknickens des Drahtes im Bereich seines proximalen Endes besteht.

Der längsverschiebliche Draht besteht in einer bevorzugten Ausführungsvariante aus wenigstens zwei Einzeldrähten, die proximal der vorgekrümmten Endabschnitte miteinander verdrillt sind.

Alternativ kann der längsverschiebliche Draht auch vergleichsweise kürzere Drahtabschnitte aufweisen und somit nicht nur mit seinem distalen Ende, sondern vollständig aus dem Lumen herauszuschieben sein. Der längsverschiebliche Draht bildet dann Implantate, die im Körper eines Patienten verbleiben können. Derartige Implantate können beispielsweise die Form kleiner Drahtkreuze haben, die durch Verdrillen zweier Drahtabschnitte in einem zentralen Abschnitt miteinander verbunden sind und sowohl an ihrem proximalen Ende als auch an ihrem distalen Ende vorgekrümmte Endabschnitte besitzen.

Zum Vorschieben eines zunächst in dem Lumen der Hohlnadel befindlichen lmplantats kann ein Schieberdraht vorgesehen sein, der sich vom proximalen Ende des Lumens her bis zu dem Implantat erstreckt und soweit vorgeschoben werden kann, dass das Implantat aus dem distalen Ende des Implantats herauszuschieben ist.

Anstelle eines einzigen Implantats können auch mehrere Implantate in Längsrichtung des Lumens magazinartig hintereinander angeordnet sein, die mittels des Schieberdrahtes einzeln aus dem Lumen herausgeschoben werden können. Dazu kann der Schieberdraht Längsmarkierungen ausweisen, die in Längsrichtung des Schieberdrahtes einen Abstand voneinander haben, der der gestreckten Länge eines jeweiligen einzelnen Implantats in dem Lumen entspricht. Dies erlaubt es beispielsweise einem Arzt, den Schieberdraht jeweils genau so weit weiter vorzuschieben, wie erforderlich ist um jeweils ein (weiteres) Implantat abzugeben.

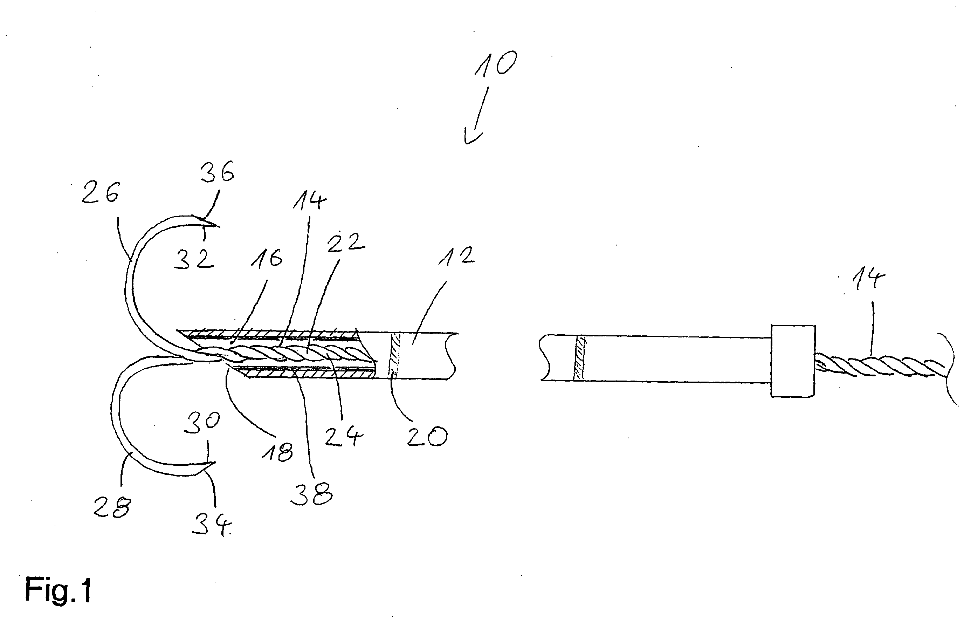

Eine Ausführungsvariante des chirurgischen Instrumentes soll nachfolgend am Beispiel eines Markierungsinstrumentes mit Bezug auf die Figur 1 beschrieben werden. Die Figur 1 zeigt das beispielhafte Markierungsinstrument in teilweise geschnittener Darstellung.

Wesentliche Bestandteile des Markierungsinstrumentes 10 sind eine Hohlnadel 12 und ein längsverschieblicher Draht in Form eines Markerdrahtes 14.

Die Hohlnadel 12 ist von einer dünnen Röhre gebildet, die bei dem Ausführungsbeispiel aus Edelstahl besteht, aber grundsätzlich auch aus einem anderen körperverträglichen Metall hoher Festigkeit gefertigt sein kann. Die Hohlnadel 12 hat einen Außendurchmesser von höchstens 1 mm und gilt damit als Feinnadel. Im Ausführungsbeispiel besitzt die Röhre einen kreisförmigen Querschnitt, andere Querschnitte sind jedoch auch denkbar. Die Hohlnadel 12 schließt ein an beiden Enden offenes Lumen 16 ein und ist im Bereich ihres distalen Endes 18 angespitzt. Auf der Außenseite der Hohlnadel 12 sind Markierungen 20 in einen Abstand von beispielsweise jeweils 1 cm voneinander vorgesehen, die es dem Arzt erlauben, die Einstechtiefe der Hohlnadel zu erfassen.

Der Markerdraht 14 ist in dem hier beschriebenen Ausführungsbeispiel aus zwei Einzeldrähten 22 und 24 gebildet, die jeweils in einem distalen Endabschnitt 26 beziehungsweise 28 vorgekrümmt sind und die proximal der distalen Endabschnitte 26 und 28 miteinander verdrillt sind. Die Vorspannung der Endabschnitte 26 und 28 ist derart, dass die Krümmung der Endabschnitte 26 und 28 im entspannten Zustand in entgegengesetzte Richtung verläuft. Im entspannten Zustand beschreiben die distalen Endabschnitte 26 und 28 jeweils einen Bogen von mehr als 180°.

Die Einzeldrähte haben im Ausführungsbeispiel jeweils einen annähernd kreisförmigen Querschnitt, können aber z.B. auch abgeflacht sein.

An ihrem jeweiligen distalen Ende 30 und 32 sind die distalen Endabschnitte 26 und 28 durch einen Anschliff 34 beziehungsweise 36 angespitzt. Die aus dem Anschliff resultierenden Anschliffflächen 34 und 36 sind so angeordnet, dass sie bei gestreckten Endabschnitten des Markerdrahtes einander zugewandt sind.

Die Endabschnitte 26 und 28 sind dann gestreckt, wenn der Markerdraht in das Lumen 16 der Hohlnadel 12 zurückgezogen wird, da der Innendurchmesser des Lumens 16 nur geringfügig größer ist als der Außendurchmesser des verdrillten Abschnittes des Markerdrahtes 14.

Damit die Endabschnitte 26 und 28 ihre vorgekrümmte Form annehmen, wenn sie in Folge des Vorschiebens des Markerdrahtes 14 in proximaler Richtung bezüglich der Hohlnadel 12 aus deren distalen Ende austreten, sind die Einzeldrähte des Markerdrahtes wenigstens teilweise aus Nitinol hergestellt. Nitinol ist eine superelastische Nickel-Titan-Legierung.

Ein wesentliches Merkmal des Markierungsinstrumentes 10 ist es, dass die Innenwand des Lumens wenigstens im Bereich des distalen Endes des Lumens 16 mit einer Gleitbeschichtung 38 versehen ist, die von einem langkettigen, biokompatiblen Wachs gebildet ist, das einen Schmelzpunkt von über 60°C aufweist. Der Schmelzpunkt von über 60° bringt den Vorteil mit sich, dass das Markierungsinstrument bei Temperaturen bis zu dieser Temperatur zu sterilisieren ist.

Mit Hilfe der Gleitbeschichtung 38 ist es möglich, den distalen Endabschnitten 26 und 28 eine derartige Vorspannung aufzuprägen, die zu einer vorteilhaften Krümmungsgeometrie führt, wenn die distalen Endabschnitte 26 und 28 aus dem Lumen 16 herausgeschoben werden und dabei in Körpergewebe eindringen. Trotz der erforderlichen Vorspannung werden dank der Gleitbeschichtung Probleme wie das Ausknicken des Markerdrahtes 14 beim Vorschieben vermieden.

## Patentansprüche

1. Chirurgisches Instrument, insbesondere Markierungsinstrument zum Markieren von Körpergewebe, insbesondere von Tumorgewebe,
mit einer Hohlnadel (12) zum Einstechen in Körpergewebe,
welche ein zum distalen Ende (18) der Hohlnadel (12) hin offenes und von einer Innenwand radial begrenztes Lumen (16) einschließt und
mit mindestens einem längsverschieblichen Element (14),
das derart längsverschieblich in dem Lumen (16) geführt ist, dass wenigstens ein distaler Endabschnitt (26; 28) des längsverschieblichen Elementes (14) aus dem offenen distalen Ende des Lumens (16) herauszuschieben ist,
**dadurch gekennzeichnet, dass** die Innenwand des Lumens (16) mindestens abschnittsweise eine Gleitbeschichtung (38) aufweist, wobei das Gleitmittel ein langkettiges biokompatibles Wachs ist, welches einen Schmelzpunkt

2. Chirurgisches Instrument nach Anspruch 1, **dadurch gekennzeichnet, dass** das längsverschiebliche Element ein Draht ist,
der wenigstens einen biegeelastischen distalen Endabschnitt (26; 28) aufweist, der im gestreckten Zustand derart elastisch vorgespannt ist, dass er sich im entspannten Zustand krümmt.

3. Chirurgisches Instrument nach Anspruch 2, **dadurch gekennzeichnet, dass** der distale Endabschnitt (26; 28) wahlweise aus dem offenen distalen Ende des Lumens (16) herauszuschieben ist und sich dabei krümmt oder in das Lumen (16) zurückzuziehen ist und dabei gestreckt wird.

4. Chirurgisches Instrument nach einem der Ansprüche 1 bis 3, **dadurch gekennzeichnet, dass** das distale Ende (30; 32) des distalen Endabschnittes (26; 28) oder die distalen Enden der distalen Endabschnitte (26; 28) angespitzt sind.

5. Chirurgisches Instrument nach einem der Ansprüche 1 bis 4, **dadurch gekennzeichnet, dass** das distale Ende des distalen Endabschnittes oder die distalen Enden der distalen Endabschnitte durch Anschleifen oder Anschneiden angespitzt sind.

6. Chirurgisches Instrument nach Anspruch 5, **dadurch gekennzeichnet, dass** ein jeweiliges distales Ende eine schräge Anschliff- oder Anschnittfläche (34; 36) aufweist, die im spitzen Winkel zu einer Mittelachse eines jeweiligen distalen Endabschnittes (26, 28) verläuft.

7. Chirurgisches Instrument nach Anspruch 5 oder 6, **dadurch gekennzeichnet, dass** eine jeweilige Anschlifffläche (34; 36) oder Anschnittfläche bei in das Lumen (16) zurückgezogenem längsverschiebliche Draht (14) nach innen weisend angeordnet ist.

8. Chirurgisches Instrument nach einem der Ansprüche 1 bis 7, **dadurch gekennzeichnet, dass** der längsverschiebliche Draht (14) ein superelastisches Material, insbesondere eine superelastische Metalllegierung wie Nitinol enthält.

9. Chirurgisches Instrument nach einem der Ansprüche 1 bis 8, **dadurch gekennzeichnet, dass** der längsverschiebliche Draht (14) im Bereich der distalen Endabschnitte derart vorgespannt ist, dass er im entspannten Zustand eine Krümmung von wenigstens 180° annimmt.

10. Chirurgisches Instrument nach einem der Ansprüche 1 bis 9, **dadurch gekennzeichnet, dass** der längsverschiebliche Draht (14) wenigstens zwei distale Endabschnitte (26; 28) besitzt, die sich im entspannten Zustand in unterschiedliche, vorzugsweise entgegengesetzte Richtung krümmen.

11. Chirurgisches Instrument nach Anspruch 10, **dadurch gekennzeichnet, dass** der längsverschiebliche Draht (14) von wenigstens zwei Einzeldrähten (22; 24) gebildet ist, die proximal der distalen Endabschnitte miteinander verdrillt sind.

12. Chirurgisches Instrument nach Anspruch 1, **dadurch gekennzeichnet, dass** der längsverschiebliche Draht die Form eines vollständig aus dem Lumen herauszuschiebenden Implantats hat.

13. Chirurgisches Instrument nach Anspruch 12, **dadurch gekennzeichnet, dass** das chirurgische Instrument einen Schieberdraht aufweist, der proximal des längsverschieblichen Drahtes längsverschieblich in dem Lumen geführt und derart ausgebildet ist, dass der längssverschiebliche Draht als Implantat mittels des Schieberdrahtes vollständig aus dem Lumen herauszuschieben ist.

## Claims

1. Surgical instrument, in particular a marking instrument for marking body tissue, in particular tumour tissue,
with a hollow needle (12) for inserting into body tissue,
which encloses a lumen (16) which is open towards the distal end (18) of the hollow needle (12) and delimited radially by an inner wall and
with at least one longitudinally displaceable element (14),
which is guided longitudinally displaceably in the lumen (16), such that at least one distal end section (26; 28) of the longitudinally displaceable element (14) is pushed out of the open distal end of the lumen (16),
**characterised in that** the inner wall of the lumen (16) has a lubricant coating (38) at least in sections, wherein the lubricant is a long-chain biocompatible wax with a melting point of over 50 °C.

2. Surgical instrument according to claim 1, **characterised in that** the longitudinally displaceable element is a wire, which comprises at least one flexible elastic distal end section (26; 28), which in the extended state is pretensioned elastically so that it curves in the relaxed state.

3. Surgical instrument according to claim 2, **characterised in that** the distal end section (26; 28) is pushed optionally out of the open distal end of the lumen (16) and thereby curves or is pulled back into the lumen (16) and thereby stretched.

4. Surgical instrument according to one of claims 1 to 3, **characterised in that** the distal end (30; 32) of the distal end section (26; 28) or the distal ends of the distal end sections (26; 28) are pointed.

5. Surgical instrument according to one of claims 1 to 4, **characterised in that** the distal end of the distal end section or the distal ends of the distal end sections are pointed by grinding or cutting.

6. Surgical instrument according to claim 5, **characterised in that** a respective distal end has an oblique ground or cut surface (34; 36) which is at an acute angle to a middle axis of a respective distal end section (26, 28).

7. Surgical instrument according to claim 5 or 6, **characterised in that** a respective ground surface (34; 36) or cut surface with the longitudinally displaceable wire (14) pulled back into the lumen (16) is arranged pointing inwards.

8. Surgical instrument according to one of claims 1 to 7, **characterised in that** the longitudinally displaceable wire (14) contains a super-elastic material, in particular a super-elastic metal alloy such as Nitinol.

9. Surgical instrument according to one of claims 1 to 8, **characterised in that** the longitudinally displaceable wire (14) is pretensioned in the region of the distal end sections, such that in the relaxed state it adopts a curvature of at least 180°.

10. Surgical instrument according to one of claims 1 to 9, **characterised in that** the longitudinally displaceable wire (14) has at least two distal end sections (26; 28), which in the relaxed state curve in different, preferably opposite directions.

11. Surgical instrument according to claim 10, **characterised in that** the longitudinally displaceable wire (14) is formed by at least two individual wires (22; 24), which are twisted together proximally of the distal end sections.

12. Surgical instrument according to claim 1, **characterised in that** the longitudinally displaceable wire has the shape of an implant to be pushed completely out of the lumen.

13. Surgical instrument according to claim 12, **characterised in that** the surgical instrument comprises a sliding wire, which is guided longitudinally displaceably in the lumen proximally of the longitudinally displaceable wire and is designed so that the longitudinally displaceable wire is completely pushed out of the lumen as an implant by means of the sliding wire.

## Revendications

1. Instrument chirurgical, en particulier, instrument de marquage pour le marquage de tissus corporels, en particulier de tissu tumoraux,
avec une aiguille creuse (12) destinée à être enfoncée dans le tissu corporel,
qui renferme une lumière (16) ouverte en direction de l'extrémité distale (18) de l'aiguille creuse (12) et délimitée radialement par une paroi intérieure et
avec au moins un élément déplaçable longitudinalement (14)
qui est guidé de manière déplaçable longitudinalement dans la lumière (16) de telle sorte qu'au moins une portion d'extrémité distale (26 ; 28) de l'élément déplaçable longitudinalement (14) peut être sortie hors de l'extrémité distale ouverte de la lumière (16),
**caractérisé en ce que** la paroi intérieure de la lumière (16) comporte, au moins par portions, un revêtement antifriction (38), dans lequel l'agent antifriction est une cire biocompatible à chaîne longue qui présente un point de fusion supérieur à 50°C.

2. Instrument chirurgical selon la revendication 1, **caractérisé en ce que** l'élément déplaçable longitudinalement est un fil,
qui comporte au moins une portion d'extrémité distale élastique (26 ; 28) qui, à l'état étiré, est précontrainte élastiquement de telle sorte qu'elle se courbe à l'état détendu.

3. Instrument chirurgical selon la revendication 2, **caractérisé en ce que** la portion d'extrémité distale (26 ; 28) peut être, au choix, soit poussée hors de l'extrémité distale ouverte de la lumière (16) et, ainsi, se courber, soit repoussée dans la lumière (16) et être ainsi étirée.

4. Instrument chirurgical selon l'une quelconque des revendications 1 à 3, **caractérisé en ce que** l'extrémité distale (30; 32) de la portion d'extrémité distale (26; 28) ou les extrémités distales des portions d'extrémité distales (26 ; 28) est aiguisée ou sont aiguisées.

5. Instrument chirurgical selon l'une quelconque des revendications 1 à 4, **caractérisé en ce que** l'extrémité distale de la portion d'extrémité distale ou les extrémités distales des portions d'extrémité distales est aiguisée ou sont aiguisées par affûtage ou lamage.

6. Instrument chirurgical selon la revendication 5, **caractérisé en ce qu'**une extrémité distale respective comporte une surface d'affûtage ou de lamage (34; 36) qui s'étend sous un angle aigu par rapport à un axe médian d'une portion d'extrémité distale respective (26 ; 28).

7. Instrument chirurgical selon la revendication 5 ou 6, **caractérisé en ce qu'**une surface d'affûtage (34 ; 36) ou surface de lamage respective est disposée dirigée vers l'intérieur lorsque le fil déplaçable longitudinalement (14) est rétracté dans la lumière (16).

8. Instrument chirurgical selon l'une quelconque des revendications 1 à 7, **caractérisé en ce que** le fil déplaçable longitudinalement (14) contient une matière super-élastique, en particulier un alliage métallique super-élastique tel que du nitinol.

9. Instrument chirurgical selon l'une quelconque des revendications 1 à 8, **caractérisé en ce que** le fil déplaçable longitudinalement (14) est précontraint dans la zone des portions d'extrémité distales de telle manière qu'il se courbe sur au moins 180° à l'état détendu.

10. Instrument chirurgical selon l'une quelconque des revendications 1 à 9, **caractérisé en ce que** le fil déplaçable longitudinalement (14) comporte au moins deux portions d'extrémité distales (26; 28) qui se courbent dans une direction différente, de préférence opposée, à l'état détendu.

11. Instrument chirurgical selon la revendication 10, **caractérisé en ce que** le fil déplaçable longitudinalement (14) est constitué par au moins deux fils individuels (22 ; 24) qui sont torsadés l'un avec l'autre à proximité des portions d'extrémité distales.

12. Instrument chirurgical selon la revendication 1, **caractérisé en ce que** le fil déplaçable longitudinalement a la forme d'un implant pouvant être complètement extrait de la lumière.

13. Instrument chirurgical selon la revendication 12, **caractérisé en ce que** l'instrument chirurgical comporte un fil coulissant qui est guidé de manière déplaçable longitudinalement dans la lumière à proximité du fil déplaçable longitudinalement et qui est conçu de telle manière que le fil déplaçable longitudinalement sous forme d'implant peut être complètement extrait de la lumière à l'aide du fil coulissant.
